# EUROPEAN PATENT APPLICATION

(11) **EP 1 918 652 A2**
(43) Date of publication of application: **07.05.2008**
(21) Application number: 07021198.2
(22) Date of filing: 30.10.2007
(51) Int. Cl.: F24F 3/16, F24F 6/04

(54) **Air bacteria removal device**

(30) Priority: 30.10.2006 JP 2006294164
(71) Applicant: Sanyo Electric Co., Ltd., Moriguchi-shi, Osaka (JP)
(72) Inventor: Sonoda, Yasuki, Ora-gun Gunma, 370-0533 (JP); Minoshima, Haruki, Saitama-shi Saitama, 336-0025 (JP); Iwama, Akifumi, Tsukuba-shi Ibaraki, 305-0861 (JP); Tateno, Ichiro, Ota-shi Gunma, 370-0311 (JP); Nakanishi, Masafumi, Ora-gun Gunma, 370-0514 (JP); Kano, Kenzo, Ora-gun Gunma, 370-0514 (JP); Ando, Akiyoshi, Yokohama-shi Kanagawa, 246-0015 (JP); Kozuka, Shinichi, Shibuya-ku Tokyo, 151-0071 (JP)
(74) Representative: Glawe, Delfs, Moll

(57) **Abstract**

An object is to provide an air bacteria removal device in which leakage of electrolytic water to the outside is eliminated as much as possible and in which maintenance can easily be performed, the air bacteria removal device comprises a main body including a water storage section having a sealed or semi-sealed structure in which the electrolytic water is stored, and a water absorption member for supplying the electrolytic water from the water storage section to an air-liquid contact member by use of a capillary phenomenon, the air-liquid contact member and the water absorption member are integrated and detachably attached to the main body, the water absorption member attached to this main body enters the water storage section, and is submerged into the electrolytic water, and the water storage section is brought into a sealed or semi-sealed state.

## Description

### BACKGROUND OF THE INVENTION

The present invention relates to an air bacteria removal device in which electrolytic water is brought into contact with passed air to remove bacteria from the air.

Heretofore, for a purpose of removal of microorganisms floating in air and the like, a bacteria removal device has been proposed in which electrolytic water mist is scattered in the air, and this electrolytic water mist is directly brought into contact with the microorganisms floating in the air to inactivate viruses and the like (e.g., see Japanese Patent Application Laid-Open No. 2002-181358).

However, there is a restriction on a space in which the electrolytic water mist can be scattered. Therefore, the above-mentioned bacteria removal device is effective in a small space in which the electrolytic water mist can be scattered, but has a problem that the effect cannot easily be exhibited in a large space such as a public facility or a vehicle.

Furthermore, the above-mentioned conventional bacteria removal device has disadvantages that a large amount of water for producing the mist is used in order to scatter the mist of the electrolytic water in the space and that the space is humidified by the mist to fog a window.

To solve the problems, the applicant has beforehand developed an air bacteria removal device of a floor mounting type including an air-liquid contact member which is arranged in a brace-like form in a housing of the floor mounting type and which is formed of a material less reactive with the electrolytic water, electrolytic water drip means constituted of a pump mechanism or the like for allowing the electrolytic water to drip down to this air-liquid contact member, and air blow means for blowing air of a bacteria removal space to the air-liquid contact member. The air sucked by the air blow means from a suction port disposed at a lower portion of the housing is brought into contact with the electrolytic water which has dripped down to the air-liquid contact member to blow the air from a blow outlet disposed at an upper portion of the housing.

However, the above-mentioned air bacteria removal device needs to be provided with a special pump mechanism or the like (electrolytic water drop means) for pumping up the produced electrolytic water to allow the water to drip down to the air-liquid contact member from the upside. Furthermore, the device needs to have a structure capable of collecting at least the electrolytic water excessively supplied to the air-liquid contact member, and hence problems have occurred that the structure is complicated, the device is enlarged and costs soar.

In addition, since dust in the air and a scale in the electrolytic water stick to the air-liquid contact member, daily maintenance such as cleaning is required, but such a structure also has a problem that it is difficult to remove the air-liquid contact member from the air bacteria removal device. Furthermore, in such a structure, sealability cannot easily be secured, and the electrolytic water might leak to the outside in a case where the device tilts. Therefore, many problems to solve are left in practically using the conventional bacteria removal device as the air bacteria removal device in a vehicle such as a car or a train.

### SUMMARY OF THE INVENTION

The present invention has been developed to solve such problems of the conventional technology, and an object thereof is to provide an air bacteria removal device in which leakage of electrolytic water to the outside is eliminated as much as possible and'in which maintenance can easily be performed.

In an air bacteria removal device of a first invention, an air-liquid contact member formed of a material less reactive with electrolytic water brings the electrolytic water into contact with passed air to remove bacteria, the device comprising: a main body including a water storage section having a sealed or semi-sealed structure in which the electrolytic water is stored; and a water absorption member which supplies the electrolytic water from the water storage section to the air-liquid contact member by use of a capillary phenomenon, characterized in that the air-liquid contact member and the water absorption member are integrated and detachably attached to the main body, the water absorption member attached to the main body enters the water storage section, and is submerged into the electrolytic water, and the water storage section is brought into a sealed or semi-sealed state.

In an air bacteria removal device of a second invention, an air-liquid contact member formed of a material less reactive with electrolytic water brings the electrolytic water into contact with passed air to remove bacteria, the device comprising: a main body including a water storage section having a sealed or semi-sealed structure in which the electrolytic water is stored; and air blow means for passing air through the air-liquid contact member, characterized in that the electrolytic water is supplied from the water storage section to the air-liquid contact member by use of a capillary phenomenon, and dust collection means and/or deodorizing means is disposed in an air path in which the air is passed through the air-liquid contact member from the air blow means.

In an air bacteria removal device of a third invention, an air-liquid contact member formed of a material less reactive with electrolytic water brings the electrolytic water into contact with passed air to remove bacteria, the device comprising: a main body including a water storage section having a sealed or semi-sealed structure in which the electrolytic water is stored; electrodes which are positioned in the water storage section and which are energized to electrochemically treat water including at least chloride ions in the water storage section, thereby producing the electrolytic water; a partition wall which separates an upper space around the electrodes from another part; and a gas release device which discharges a gas from the space on an inner side of the partition wall, characterized in that the electrolytic water is supplied from the water storage section to the air-liquid contact member by use of a capillary phenomenon.

In an air bacteria removal device of a fourth invention, an air-liquid contact member formed of a material less reactive with electrolytic water brings the electrolytic water into contact with passed air to remove bacteria, the device comprising: a main body including a water storage section having a sealed or semi-sealed structure in which the electrolytic water is stored; electrodes which are positioned in the water storage section and which are energized to electrochemically treat water in the water storage section, thereby producing the electrolytic water; and a gas release device which discharges a gas from the water storage section, characterized in that the electrolytic water is supplied from the water storage section to the air-liquid contact member by use of a capillary phenomenon, and an outlet of the gas release device is arranged in the vicinity of the air-liquid contact member.

In an air bacteria removal device of a fifth invention, an air-liquid contact member formed of a material less reactive with electrolytic water brings the electrolytic water into contact with passed air to remove bacteria, the device comprising: a main body including a water storage section having a sealed or semi-sealed structure in which the electrolytic water is stored; electrodes which are positioned in the water storage section and which are energized to electrochemically treat water including at least chloride ions in the water storage section, thereby producing the electrolytic water; a water supply tank which is detachably attached to the main body and which refills the water storage section with the water including at least the chloride ions; and a valve device which prohibits water supply from the water supply tank in a case where the main body tilts, characterized in that the electrolytic water is supplied from the water storage section to the air-liquid contact member by use of a capillary phenomenon.

In an air bacteria removal device of a sixth invention, an air-liquid contact member formed of a material less reactive with electrolytic water brings the electrolytic water into contact with passed air to remove bacteria, the device comprising: a main body including a water storage section having a sealed or semi-sealed structure in which the electrolytic water is stored; electrodes which are positioned in the water storage section and which are energized to electrochemically treat water including at least chloride ions in the water storage section, thereby producing the electrolytic water; a water supply tank which is detachably attached to the main body and which refills the water storage section with the water including at least the chloride ions; and air blow means for passing air through the air-liquid contact member, characterized in that the electrolytic water is supplied from the water storage section to the air-liquid contact member by use of a capillary phenomenon, and the air is introduced from the outside by the air blow means, brought into contact with the air-liquid contact member and then discharged to the outside.

In an air bacteria removal device of a seventh invention, the sixth invention further comprises: a water absorption member having one end portion which comes in contact with the air-liquid contact member and the other end portion submerged into the electrolytic water in the water storage section to supply the electrolytic water to the air-liquid contact member by use of the capillary phenomenon ; and a float valve device which controls the water supply from the water supply tank, characterized in that when a water level of the electrolytic water rises above a water level at which at least the other end portion of the water absorption member is submerged into the electrolytic water in the water storage section, the float valve device prohibits the water supply from the water supply tank.

In an air bacteria removal device of an eighth invention, the sixth invention or the seventh invention is characterized in that the air-liquid contact member is arranged on one end portion of the main body, the water supply tank is arranged on the other end portion of the main body, the water storage section is constituted from the one end portion to the other end portion of the main body, the electrodes are arranged in the water storage section between the water supply tank and the air-liquid contact member, the air blow means is arranged on the main body between the air-liquid contact member and the water supply tank, and air is sucked from an axial direction around which the air blow means rotates to discharge the air in a radial direction.

In an air bacteria removal device of a ninth invention, any one of the first to eighth inventions is characterized in that the air-liquid contact member is hydrophilically treated.

According to the first invention, in the air bacteria removal device, the air-liquid contact member formed of the material less reactive with the electrolytic water brings the electrolytic water into contact with the passed air to remove the bacteria. The device comprises the main body including the water storage section having the sealed or semi-sealed structure in which the electrolytic water is stored; and the water absorption member which supplies the electrolytic water from the water storage section to the air-liquid contact member by use of the capillary phenomenon. Therefore, the electrolytic water in the water storage section is sucked up by the water absorption member by use of the capillary phenomenon, quickly supplied to the air-liquid contact member, and brought into contact with the passed air by the air-liquid contact member, so that the bacteria can efficiently be removed from the air.

Moreover, the air-liquid contact member and the water absorption member are integrated and detachably attached to the main body, the water absorption member attached to the main body enters the water storage section, and is submerged into the electrolytic water, and the water storage section is brought into the sealed or semi-sealed state. Therefore, sealability of the water storage section can be secured, and the water does not easily leak from the structure. Especially, such a structure is more effective in a case where the air bacteria removal device is used on conditions that the device tilts, for example, during running along a slope in a case where the air bacteria removal device is mounted on a car.

Furthermore, according to the present invention, the air-liquid contact member and the water absorption member can freely be detached, dust from the passed air and a scale in the electrolytic water attached to the air-liquid contact member and the water absorption member can be cleaned, and the members can easily be mounted on the main body after the cleaning. In consequence, daily maintenance can easily be performed.

According to the second invention, the air-liquid contact member formed of the material less reactive with electrolytic water brings the electrolytic water into contact with the passed air to remove the bacteria, and the device comprises the main body including the water storage section having the sealed or semi-sealed structure in which the electrolytic water is stored, and the air blow means for passing the air through the air-liquid contact member, and the electrolytic water is supplied from the water storage section to the air-liquid contact member by use of the capillary phenomenon. Therefore, the electrolytic water in the water storage section'is quickly supplied to the air-liquid contact member by use of the capillary phenomenon, and brought into contact with the passed air by the air-liquid contact member, so that the bacteria can efficiently be removed from the air.

Moreover, the water storage section in which the electrolytic water is stored is brought into the sealed or semi-sealed structure, so that sealability of the water storage section can be secured, and the water does not easily leak from the structure. Especially, such a structure is more effective in a case where the air bacteria removal device is used on conditions that the device tilts, for example, during running along a slope in a case where the air bacteria removal device is mounted on a car.

Furthermore, since the dust collection means and/or the deodorizing means is disposed in the air path in which the air is passed through the air-liquid contact member from the air blow means. Therefore, sticking of dust to the air-liquid contact member can be eliminated or inhibited by the dust collection means. The deodorizing means can deodorize the air.

According to the third invention, the air-liquid contact member formed of the material less reactive with the electrolytic water brings the electrolytic water into contact with the passed air to remove the bacteria. The air bacteria removal device comprises the main body including the water storage section having the sealed or semi-sealed structure in which the electrolytic water is stored, the electrodes which are positioned in the water storage section and which are energized to electrochemically treat the water including at least the chloride ions in the water storage section, thereby producing the electrolytic water, the partition wall which separates the upper space around the electrodes from the other part, and the gas release device which discharges the gas from the space on the inner side of the partition wall, and the electrolytic water is supplied from the water storage section to the air-liquid contact member by use of the capillary phenomenon. Therefore, the electrolytic water in the water storage section is quickly supplied to the air-liquid contact member by use of the capillary phenomenon, and brought into contact with the passed air by the air-liquid contact member, so that the bacteria can efficiently be removed from the air.

Moreover, the water storage section in which the electrolytic water is stored is brought into the sealed or semi-sealed structure, so that sealability of the water storage section can be secured, and the water does not easily leak from the structure. Especially, such a structure is more effective in a case where the air bacteria removal device is used on conditions that the device tilts, for example, during running along a slope in a case where the air bacteria removal device is mounted on a car.

Furthermore, the gas generated in the electrodes can smoothly trapped and discharged by the partition wall and the gas release device. Therefore, a disadvantage that a pressure in the water storage section increases owing to the generated gas can be avoided in advance.

According to the fourth invention, the air-liquid contact member formed of'the material less reactive with electrolytic water brings the electrolytic water into contact with the passed air to remove the bacteria. The air bacteria removal device comprises the main body including the water storage section having the sealed or semi-sealed structure in which the electrolytic water is stored, the electrodes which are positioned in the water storage section and which are energized to electrochemically treat the water in the water storage section, thereby producing the electrolytic water, and the gas release device which discharges the gas from the water storage section, and the electrolytic water is supplied from the water storage section to the air-liquid contact member by use of the capillary phenomenon. Therefore, since the electrolytic water is quickly supplied from the water storage section to the air-liquid contact member by use of the capillary phenomenon, the electrolytic water in the water storage section can quickly be supplied to the air-liquid contact member by use of the capillary phenomenon, and brought into contact with the passed air by the air-liquid contact member, so that the bacteria can efficiently be removed from the air. Furthermore, the water storage section in which the electrolytic water is stored is brought into the sealed or semi-sealed structure, so that sealability of the water storage section can be secured, and the water does not easily leak from the structure.

In addition, the gas release device can discharge a gas generated in the electrodes from the water storage section. Especially, the outlet of the gas release device is arranged in the vicinity of the air-liquid contact member. Therefore, even when the air bacteria removal device tilts and the electrolytic water in the water storage section enters the gas release device, the water can be discharged from the outlet of the gas release device to the air-liquid contact member, so that a disadvantage that the electrolytic water leaks to the outside can be prevented as much as possible. In consequence, the present invention is especially effective in a case where the air bacteria removal device is used on conditions that the device tilts, for example, during running along a slope in a case where the air bacteria removal device is mounted on a car.

According to the fifth invention, the air-liquid contact member formed of the material less reactive with the electrolytic water brings the electrolytic water into contact with the passed air to remove the bacteria. The air bacteria removal device comprises the main body including the water storage section having the sealed or semi-sealed structure in which the electrolytic water is stored, the electrodes which are positioned in the water storage section and which are energized to electrochemically treat the water including at least the chloride ions in the water storage section, thereby producing the electrolytic water, the water supply tank which is detachably attached to the main body and which refills the water storage section with the water including at least the chloride ions, and the valve device which prohibits the water supply from the water supply tank in a case where the main body tilts, and the electrolytic water is supplied from the water storage section to the air-liquid contact member by use of the capillary phenomenon. Therefore, the electrolytic water in the water storage section is quickly supplied to the air-liquid contact member by use of the capillary phenomenon, and brought into contact with the passed air by the air-liquid contact member, so that the bacteria can efficiently be removed from the air. Moreover, the water storage section in which the electrolytic water is stored is brought into the sealed or semi-sealed structure, so that sealability of the water storage section can be secured, and the water does not easily leak from the structure.

Especially, when the main body tilts, the valve device prohibits the water supply from the water supply tank. Therefore, it is possible to securely avoid a disadvantage that when a water supply tank side tilts high and a water level at the outlet of the water supply tank drops, the water is excessively supplied from the water supply tank to the water storage section. In consequence, the present invention is especially effective in a case where the air bacteria removal device is used on conditions that the device tilts, for example, during running along a slope in a case where the air bacteria removal device is mounted on a car.

According to the sixth invention, the air-liquid contact member formed of the material less reactive with the electrolytic water brings the electrolytic water into contact with the passed air to remove the bacteria. The air bacteria removal device comprises the main body including the water storage section having the sealed or semi-sealed structure in which the electrolytic water is stored, the electrodes which are positioned in the water storage section and which are energized to electrochemically treat the water including at least the chloride ions in the water storage section, thereby producing the electrolytic water, the water supply tank which is detachably attached to the main body and which refills the water storage section with the water including at least the chloride ions, and the air blow means for passing the air through the air-liquid contact member. The electrolytic water is supplied from the water storage section to the air-liquid contact member by use of the capillary phenomenon, and the air is introduced from the outside by the air blow means, brought into contact with the air-liquid contact member and then discharged to the outside. Therefore, the electrolytic water in the water storage section can quickly be supplied to the air-liquid contact member by use of the capillary phenomenon, and brought into contact with the passed air by the air-liquid contact member, so that the bacteria can efficiently be removed from the air. Furthermore, the water storage section in which the electrolytic water is stored is brought into the sealed or semi-sealed structure, so that sealability of the water storage section can be secured, and the water does not easily leak from the structure. Especially, such a structure is more effective in a case where the air bacteria removal device is used on conditions that the device tilts, for example, during running along a slope in a case where the air bacteria removal device is mounted on a car.

Furthermore, the electrolytic water in the water storage section is supplied to the air-liquid contact member by use of the capillary phenomenon, whereby a structure capable of collecting the electrolytic water supplied to the air-liquid contact member is not required, unlike a conventional circulation type in which the electrolytic water brought into contact with the air-liquid contact member is returned to the water storage section. Therefore, the structure can be simplified to miniaturize the device.

According to the seventh invention, the sixth invention further comprises the water absorption member having the one end portion which comes in contact with the air-liquid contact member and the other end portion submerged into the electrolytic water in the water storage section to supply the electrolytic water to the air-liquid contact member by use of the capillary phenomenon, and the float valve device which controls the water supply from the water supply tank. When the water level of the electrolytic water rises above the water level at which at least the other end portion of the water absorption member is submerged into the electrolytic water in the water storage section, the float valve device prohibits the water supply from the water supply tank. Therefore, the water absorption member can constantly be submerged into the electrolytic water. In consequence, the supply of the electrolytic water to the air-liquid contact member can firmly be secured.

According to the eighth invention, in the sixth invention or the seventh invention, the air-liquid contact member is arranged on the one end portion of the main body, the water supply tank is arranged on the other end portion of the main body, the water storage section is constituted from the one end portion to the other end portion of the main body, the electrodes are arranged in the water storage section between the water supply tank and the air-liquid contact member, the air blow means is arranged on the main body between the air-liquid contact member and the water supply tank, and the air is sucked from the axial direction around which the air blow means rotates to discharge the air in the radial direction, so that the whole device can be slimmed. In consequence, while the miniaturization of the device is achieved, it is possible to secure a series of operations of sucking the outside air to blow the air to the air-liquid contact member, refilling the water storage section with the water including at least the chloride ions from the water supply tank, and electrolyzing the water to supply the water to the air-liquid contact member.

According to the ninth invention, in any one of the first to eighth inventions, the air-liquid contact member is hydrophilically treated, so that hydrophilicity with respect to the electrolytic water can be improved. In consequence, an electrolytic water retention property of the air-liquid contact member is kept, and the air passed through the air-liquid contact member can be kept in contact with the electrolytic water for a long time.

### BRIEF DESCRIPTION OF THE DRAWINGS

FIG. 1 is an appearance diagram of an air bacteria removal device according to one embodiment of the present invention;
FIG. 2 is a vertical side view of the air bacteria removal device of FIG. 1 as viewed from one surface side (front surface side);
FIG. 3 is a vertical side view of the air bacteria removal device of FIG. 2 as viewed from an opposite side (back surface side);
FIG. 4 is an inner constitution diagram of the air bacteria removal device in a state in which a main body cover of FIG. 1 is removed;
FIG. 5 is an exploded diagram of the air bacteria removal device of FIG. 4;
FIG. 6 is a partially enlarged view of the air bacteria removal device of FIG. 3; and
FIG. 7 is a partially enlarged view of the air bacteria removal device of FIG. 5.

### DETAILED DESCRIPTION OF THE PREFERRED EMBODIMENT

The present invention relates to an air bacteria removal device in which an air-liquid contact member brings electrolytic water into contact with passed air to remove microorganisms floating in air and the like, and the device has been developed in order to easily realize maintenance of the air-liquid contact member while eliminating a disadvantage that the electrolytic water leaks to the outside as much as possible. An object to provide an air bacteria removal device in which the water leakage is eliminated as much as possible and in which maintenance can easily be performed is achieved by a device including a main body having a water storage section of a sealed or semi-sealed structure in which the electrolytic water is stored, and a water absorption member for supplying the electrolytic water from the water storage section to the air-liquid contact member by use of a capillary phenomenon. The air-liquid contact member and the water absorption member are integrated and detachably attached to the main body, this water absorption member attached to the main body enters the water storage section, and is submerged into the electrolytic water, and the water storage section is brought into a sealed or semi-sealed state. According to the present invention, especially the structure can be realized in which sealability of the water storage section is secured so that water does not easily leak. Therefore, the present invention is preferably mounted in a compartment of an automobile such as a car or a bus and a compartment of a train. An embodiment of the present invention will hereinafter be described in detail with reference to the drawings.

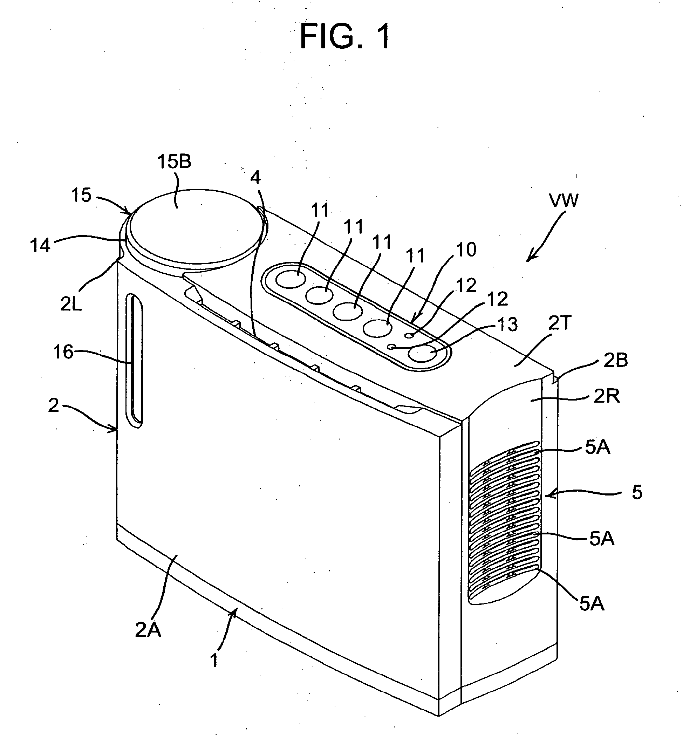

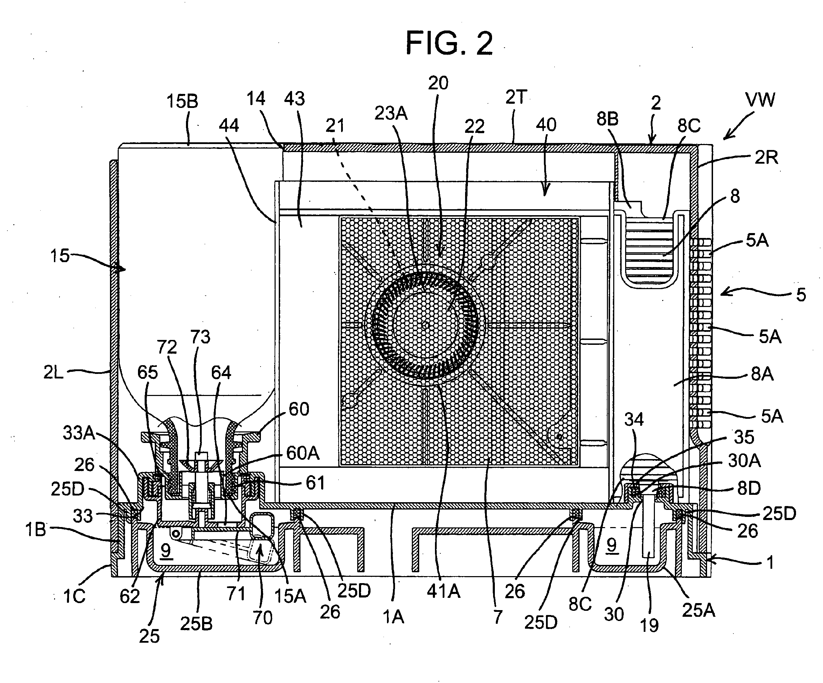

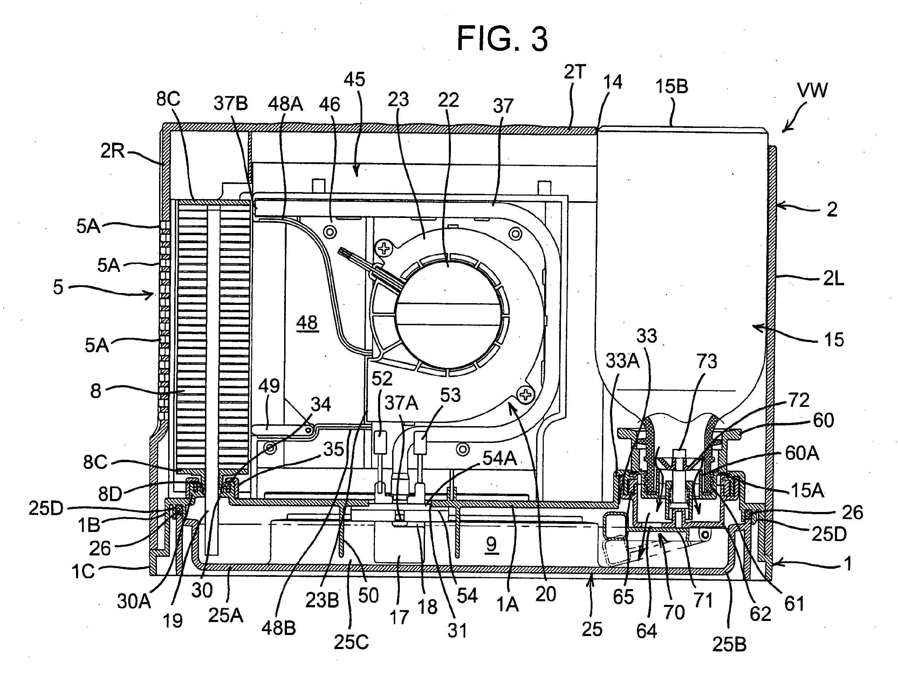

FIG. 1 is an appearance diagram of an air bacteria removal device VW according to one embodiment of the present invention, FIG. 2 is a diagram of a vertical side surface view of the air bacteria removal device VW of FIG. 1 as viewed from one surface side (front surface side), and FIG. 3 is a diagram of a vertical side surface of the device VW of FIG. 1 as viewed from another surface side (back surface side). In the drawings, reference numeral 1 is a main body of the air bacteria removal device VW according to the present embodiment, and 2 is a main body cover which is attached to the main body 1 and which covers a bacteria removal element 8, a filter 7, an air blower 20 and the like mounted on the main body 1 as described later. The main body cover 2 includes a top plate 2T and four panels (a front panel 2A, a back panel 2B, a side panel 2R and a side panel 2L) extending in a vertical direction from an outer peripheral edge of this top plate 2T.

On an upper surface of the top plate 2T, an operating section 10 is formed which includes a plurality of (four in the embodiment) operation switches 11 for control, display means 12 constituted of a plurality of (two in the embodiment) LED lamps, and a power switch 13. Moreover, a suction port 4 for sucking air in a room as a bacteria removal space into the air bacteria removal device VW (into the main body cover 2) is formed between one end portion (a front end portion in FIG. 1) of the top plate 2T and an upper end portion of the front panel 2A. The side panel 2R positioned on one end side (on a right end side of the front panel 2A and the back panel 2B in FIGS. 1 and 2) of the front panel 2A and the back panel 2B is provided with an exhaust port 5 for discharging the air from which bacteria has been removed by the bacteria removal element 8 from the air bacteria removal device VW (from the room as the bacteria removal space). This exhaust port 5 is constituted of a large number of small holes 5A ... formed at the side panel 2R along a vertical direction thereof.

Moreover, on the other end side (the side panel 2L side) of the top plate 2T of the main body cover 2, a hole 14 extending through the top plate 2T in the vertical direction (an upward/downward direction) is formed. This hole 14 is a hole for a water supply tank 15. When the main body cover 2 is attached to the main body 1, an upper surface 15B of the water supply tank 15 is positioned in this hole 14. Furthermore, the front panel 2A of the main body cover 2 is provided with a water amount confirming window 16 for confirming a water level of water (tap water as one example of water including at least chloride ions) stored in the water supply tank 15. With this window 16 for confirming the water amount, the amount of the water in the water supply tank 15 can be confirmed without removing the main body cover 2.

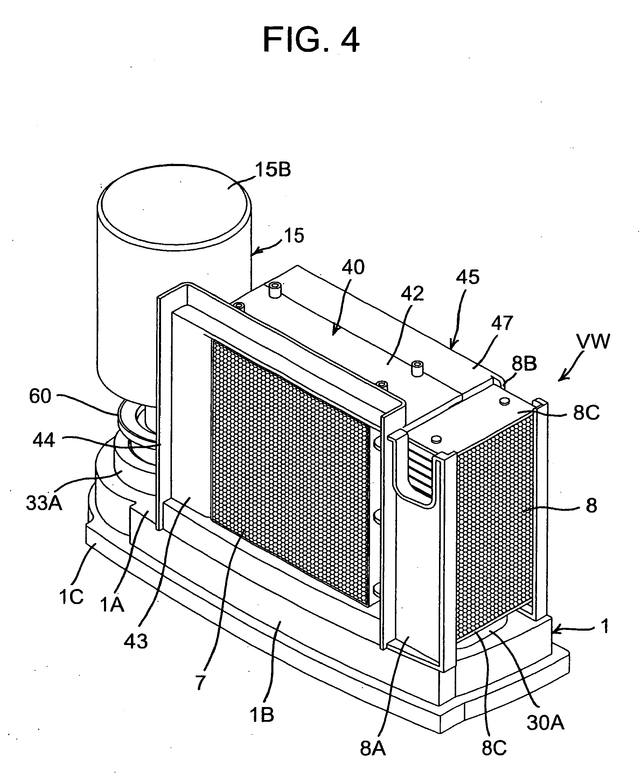

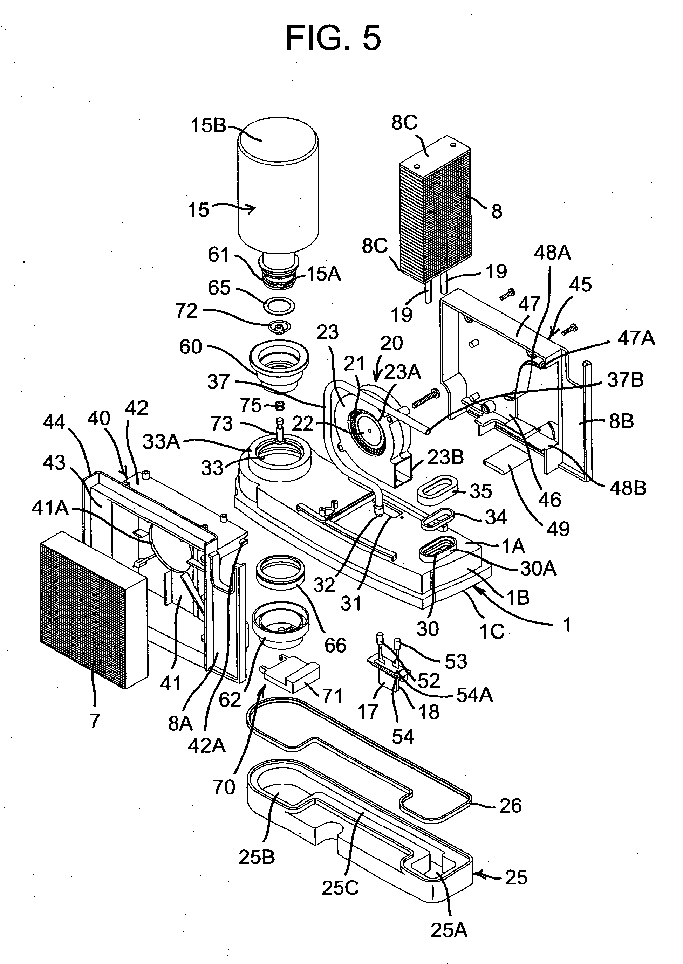

Next, an inner constitution of the air bacteria removal device VW will be described with reference to FIGS. 2 to 5. FIG. 4 shows an inner constitution diagram of the air bacteria removal device VW in a state in which the main body cover 2 is removed from the main body 1, and FIG. 5 shows an exploded diagram of the air bacteria removal device VW of FIG. 4, respectively.

The main body 1 of the air bacteria removal device VW is a frame body including a receiving plane 1A and a wall surface 1B extending in the vertical direction (a downward direction) from an outer peripheral edge of this receiving plane 1A, and a lower end of the wall surface 1B is provided with an expanded portion 1C expanded externally from the wall surface 1B. An outer periphery of the wall surface 1B is formed so as to be substantially the same as an inner periphery of the main body cover 2, and an outer periphery of the expanded portion 1C is set so as to be larger than that of the wall surface. Moreover, when the main body cover 2 is attached to the main body 1, an inner surface of a wall surface tip end (a lower end) of the main body cover 2 abuts on the wall surface 1B of the main body 1, whereby the main body cover 2 is held by the wall surface 1B of the main body 1 at the upper surface of the expanded portion 1C.

One end portion, the other end portion and the center between the one end portion and the other end portion of the receiving plane 1A of the main body 1 are provided with communication holes 30, 31, 32 and 33 which extend through the receiving plane in the vertical direction (the upward/downward direction). The communication hole 30 formed at one end portion of the receiving plane 1A is a hole for attaching the bacteria removal element 8 to one end portion of the main body 1, and an engagement portion 30A raised upwards from the receiving plane 1A is formed at the periphery of the communication hole. The communication hole 33 formed at the other end portion of the receiving plane 1A is a hole for attaching the water supply tank 15 to the other end portion of the main body 1, and an engagement portion 33A raised upwards from the receiving plane 1A is also formed at the periphery of this communication hole 33. The communication holes 31 and 32 at the center of the receiving plane 1A are formed adjacent to each other, the communication hole 31 is disposed on a wall surface 1B side, and the communication hole 32 is disposed on a front panel 2A side. Electrodes 17, 18 described later are attached to the communication hole 31, and one end of an air passing tube 37 described later is similarly connected to the communication hole 32.

Moreover, the bottom surface (the lower surface) of the receiving plane 1A is provided with an engagement portion 25D for attaching an electrolytic tank tray 25 to the main body 1. This electrolytic tank tray 25 is a longitudinal water receiving member including the side of one end portion 25A having a space in which a sufficient capacity is secured, the side of the other end portion 25B in which a sufficient capacity is similarly secured, and the center 25C which allows the one end portion 25A side and the other end portion 25B side to communicate with each other and whose one side (front side) is largely bored and which has a small inner capacity and a small shape.

An opening edge of an upper end of the electrolytic tank tray 25 is fitted into the engagement portion 25D formed at the bottom surface of the receiving plane 1A of the main body 1. Specifically, in a state in which a packing 26 as a seal member is attached to the whole periphery of the opening edge of the upper end of the electrolytic tank tray 25, the electrolytic tank tray 25 is inserted into the wall surface 1B from the downside of the expanded portion 1C of the main body 1. Then, when the electrolytic tank tray 25 is inserted into a predetermined attachment position, the upper end opening fits into the engagement portion 25D, whereby the electrolytic tank tray 25 is attached to the main body 1. In a state in which the electrolytic tank tray 25 is attached to the main body 1, the upper end opening is closed with the receiving plane 1A of the main body 1 in a sealed manner, and a water storage tank 9 having a sealed or semi-sealed structure is formed in this electrolytic tank tray 25. Then, electrolytic water described later is stored in this water storage tank 9. In the present embodiment, in a state in which the packing 26 is attached to the whole periphery of the upper end opening edge of the electrolytic tank tray 25, the tray is closed with the receiving plane 1A of the main body in the sealed manner, and the communication holes 30, 31 and 33 formed at the receiving plane 1A are closed with a water absorption member 19, the electrodes 17, 18 and the water supply tank 15, respectively, but since the air passing tube 37 is connected to the communication hole 32, not a completely sealed structure but a semi-sealed structure is constituted.

However, since the front surface side of the center of the electrolytic tank tray 25 is largely bored, and has a small inner capacity and a small shape as described above, the capacity of the electrolytic water to be stored at the center of the water storage tank 9 is necessarily reduced. Since the bacteria removal element 8 is attached to the center in the water storage tank 9, that is, one end portion of the main body 1, and the water supply tank 15 is attached to the other end portion as described above, a pair of electrodes 17, 18 (an electrolytic unit) described above are disposed in the water storage tank 9 between this bacteria removal element 8 and the water supply tank 15. The electrodes 17, 18 electrochemically treat (electrolyze) the tap water stored in the water storage tank 9 to produce the electrolytic water. Specifically, the electrodes 17, 18 are energized from a power supply to electrolyze the tap water in the water storage tank 9 and to produce electrolytic water including active oxygen species, and upper ends of the electrodes are connected to electrode terminals 52, 53. The electrode terminals 52, 53 are connected to a power supply (not shown), and the electrodes 17, 18 are energized from this power supply via the electrode terminals 52, 53.

Here, the active oxygen species include oxygen molecules having oxidation activity higher than that of usual oxygen, and related substances. It is assumed that active oxygen in a so-called narrow sense, for example, super oxide anion, singlet oxygen, hydroxyl radical or hydrogen peroxide, and active oxygen in a so-called broad sense, for example, ozone or hypohalogen acid are included.

The electrodes 17, 18 are electrode plates each including a base made of titanium (Ti) and a coat layer of iridium (Ir) or platinum (Pt). As a current value to be applied to these electrodes 17, 18, assuming that a current density is 20 milliamperes (mA)/square centimeter (cm²), a predetermined floating residual chlorine concentration (e.g., 1 milligram (mg)/liter (1) is produced.

When the tap water is energized by the electrodes 17, 18, the following reaction occurs in a cathode electrode:

4H⁺+4e⁻+(40H⁻) → 2H₂+(4OH⁻), and

the following reaction occurs in an anode electrode:

2H₂O → 4H⁺+O₂+4e⁻.

Moreover, chloride ions (added beforehand to the tap water) included in water react as follows:

2Cl⁻ → Cl₂+2e⁻.

Furthermore, Cl₂ reacts with water as follows:

Cl₂+H₂O → HClO+HCl.

As represented by the above chemical formulas, a gas such as hydrogen and oxygen is produced by the electrochemical treatment using the electrodes 17, 18 as described above.

Furthermore, in this constitution, when the electrodes 17, 18 are energized, sterile water (the electrolytic water) including hypochlorous acid (HClO) having large germicidal power can be produced. Especially, the center in the water storage tank 9 in which the electrodes 17, 18 are arranged has a small inner capacity and a narrow shape as described above, and a small capacity of tap water is stored, so that highly concentrated electrolytic water (i.e., having a high concentration of hypochlorous acid) can be produced by electrolysis of the electrodes 17, 18.

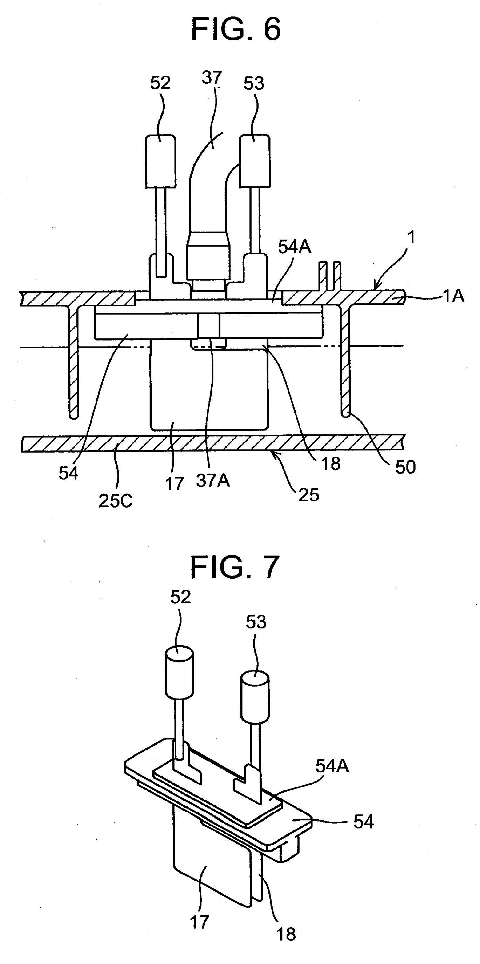

As shown in FIGS. 6 and 7, peripheries of the electrodes 17, 18 are fixed to an electrode fixing plate 54 so that the surfaces of both electrode plates are arranged so as to face each other with a predetermined space disposed therebetween. An upper end of this electrode fixing plate 54 is provided with a protruding portion 54A having substantially the same shape as that of the communication hole 31 of the main body 1, and the protruding portion 54A engages with an inner surface of the communication hole 31 to attach the plate to the main body 1. Specifically, the electrode fixing plate 54 to which the electrodes 17, 18 have been secured is inserted into the communication hole 31 from a bottom surface side (downside) of the receiving plane 1A, and the protruding portion 54A formed at the upper end is then fitted into an inner wall of the communication hole 31, whereby the protruding portion 54A is held at an inner wall surface of the communication hole 31 to attach the plate to the main body 1.

At this time, the electrodes are attached to the water storage tank 9 so that circulation of the electrolytic water in the water storage tank 9 is not disturbed, that is, so that the tap water to be supplied into the water storage tank 9 from the other end portion side (the water supply tank 15) of the main body 1 is passed through the electrodes 17, 18, and a plate surface of both the electrodes 17, 18 disposed so as to face each other so that the electrolytic water produced by the electrodes 17, 18 smoothly flows to one end portion side (the bacteria removal element 8) is disposed in parallel with a flow direction of the electrolytic water in the water storage tank 9 flowing from the other end side toward the one end side. In a state in which the electrodes 17, 18 are attached to the main body 1, at least tip ends (lower ends) of the electrodes are submerged into the electrolytic water in the water storage tank 9.

On the other hand, the air blower 20 is disposed on the center of the main body 1, that is, above the center of the water storage tank 9 in which the electrodes 17, 18 are arranged as described above. The air blower 20 is air blow means for passing air through the bacteria removal element 8, and is constituted so that air is introduced from the outside (the room), supplied to the bacteria removal element 8, brought into contact with the element and then discharged to the outside. The air blower 20 includes an air blowing fan 21, a fan motor 22 disposed at the axial center of rotation of the air blowing fan 21 and configured to rotate and drive the'air blowing fan 21, and a fan casing 23 in which a fan air suction port 23A is formed at the center on one surface side (a front surface side in FIG. 2) where the front panel 2A is positioned and in which a fan exhaust port 23B is formed at a lower portion on one end side (a right end side in FIG. 2) where the side panel 2R is positioned. The air blower 20 of the present embodiment is constituted of a sirocco fan for sucking air from the direction of an axis around which the air blowing fan 21 rotates to discharge the air in a radial direction.

The air blower 20 is arranged so that the fan air suction port 23A is positioned on the front panel 2A side and the fan exhaust port 23B is positioned on the side panel 2R side, and the air blower is attached to the upper surface of the center of the receiving plane 1A of the main body 1 in a state in which an outer periphery of the air blower is surrounded with a fixing plate (a first fixing plate 40 and a second fixing plate 45).

The fixing plate includes the first fixing plate 40 positioned on the one surface side (the front surface side) of the air blower 20, and the second fixing plate 45 positioned on the other surface side (the back surface side). As shown in FIG. 5, the first fixing plate 40 is constituted of an attachment section 44 including a flat surface 41 disposed on the one surface side (the front surface side) of the air blower 20, a peripheral wall portion 42 extending from an outer peripheral edge of the flat surface 41 on a air blower 20 side, a holding surface 8A which extends horizontally to the flat surface 41 from one end side (the right end side in FIG. 5) of the flat surface 41 and which fixes the bacteria removal element 8, and a fixing member 43 which rises further upwards from the upper edge of the flat surface 41 and whose opposite ends (left and right ends shown in FIG. 5) are bent substantially at right angles on a side opposite to the air blower 20 and in which the filter 7 is to be attached. In the flat surface 41 of the first fixing plate 40, a circular hole 41A is formed at a position corresponding to the fan air suction port 23A formed at the fan casing 23 of the air blower 20. A tip end of the peripheral wall portion 42 positioned above the flat surface 41 is provided with a concave portion 42A to be fitted into a convex portion 47A formed at the second fixing plate 45 described later.

The second fixing plate 45 includes a flat surface 46 disposed on the other surface side (the back surface side) of the air blower 20, a peripheral wall portion 47 extending from an outer peripheral edge of the flat surface 46 on the air blower 20 side, and a holding surface 8B which extends horizontally to the flat surface 46 from one end side (the right end side in FIG. 5) of the flat surface 46 and which fixes the bacteria removal element 8. On the one end side of a lower portion of the peripheral wall portion 47 positioned above the flat surface 46, the convex portion 47A to be engaged with the concave portion 42A is formed. Over one end side (a left end side in FIG. 3, a right end side in FIG. 5, i.e., a bacteria removal element 8 side) of the side (the air blower 20 side) on which the peripheral wall portion 47 of the flat surface 46 extends, the one end side constituting the bacteria removal element 8 side from a portion where the fan exhaust port 23B is positioned in a case where the air blower 20 is arranged in the fixing plates 40, 45, dividing plates 48A, 48B are attached which form an air duct 48 which supplies the air discharged from the fan exhaust port 23B to the bacteria removal element 8. Furthermore, a fin 49 for adjusting a flow direction of the air discharged from the fan exhaust port 23B is attached to the attached lower dividing plate 48B.

Furthermore, to mount the air blower 20 on the main body 1, first on one end side of the air blower 20, the hole 41A formed at the flat surface 41 and the fan air suction port 23A of the fan casing 23 of the air blower 20 are aligned to arrange the first fixing plate 40. Then, the second fixing plate 45 is arranged from the other end side of the air blower 20. At this time, when the tip end of the peripheral wall portion 47 of the second fixing plate 45 is arranged so as to abut on the tip end of the peripheral wall portion 42 of the first fixing plate 40, the convex portion 47A of the flat surface 46 is fitted into the concave portion 42A formed above the peripheral wall portion 42, whereby the air blower 20 is surrounded in a space formed by the fixing plates 40, 45. Then, in this state, both the fixing plates 40, 45 are fixed with screws from the back surface side of the second fixing plate 45, and further both the fixing plates 40, 45 are fixed to the main body 1 with screws or the like, so that the air blower can be mounted on the main body 1.

As described above, the filter 7 is attached to the attachment section 44 on the front surface side of the first fixing plate 40 via the fixing member 43. This filter 7 is means having a dust collecting function and/or a deodorizing function, and in the present embodiment, the filter 7 having both of the dust collecting function and the deodorizing function is used. The filter 7 is disposed at a position on the windward side of the bacteria removal element 8 in a path of air to be passed through the bacteria removal element 8 from the air blower 20. That is, the filter 7 of the present embodiment is detachably attached to the inside of the fixing member 43 of the attachment section 44 of the first fixing plate 40 on the windward side of the air blower 20.

Moreover, in a state in which the main body cover 2 is attached to the main body 1, the filter 7 is arranged with a predetermined space from the front panel 2A on an inner side of the front panel 2A, and the suction port 4 is positioned on an upper front surface side of the filter 7, that is, on the front panel side. Therefore, when the air blower 20 is operated, the air sucked from the suction port 4 passes through a gap formed between the front panel 2A and the filter 7, passes through the filter 7 disposed at the front surface of the air blower 20, and is then sucked into the air blower 20.

In addition, the bacteria removal element 8 is an air-liquid contact member which brings the electrolytic water stored in the water storage tank 9 into contact with the passed air from the air blower 20, and is arranged on one end portion of the main body 1. The bacteria removal element 8 of the present embodiment is a filter member having a filter structure in which a broad air-liquid contact area is secured and in which water can be held and which is not easily clogged. That is, the bacteria removal element 8 is formed into the whole honeycomb shape by bonding a material bent in a corrugated form and a flat-plate-like material. In these materials, there is used a material which is less reactive with the electrolytic water, that is, less deteriorated by the electrolytic water, for example, a polyolefin-based resin (a polyethylene resin, a polypropylene resin or the like), a PET (polyethylene, terephthanol) resin, a vinyl chloride resin, a fluorine-based resin (PTFE, PFA, ETFE or the like), a cellulose-based material or a ceramic-based material. When the bacteria removal element 8 is formed of the material less reactive with the electrolytic water in this manner, durability of the bacteria removal element 8 can be improved. The bacteria removal element 8 is subjected to a hydrophilic treatment to improve hidrophilicity to the electrolytic water, whereby a water retaining property (wettability) of the bacteria removal element 8 with respect to the electrolytic water is improved, and the air passed through the bacteria removal element 8 can be brought into contact with the electrolytic water for a long time. Furthermore, plate members having excellent resistance to corrosion are attached to an upper surface and a bottom surface of this bacteria removal element 8.

Furthermore, this bacteria removal element 8 is integrally provided with two cylindrical water absorption rods 19. The water absorption rods 19, 19 are water absorption members for sucking the electrolytic water to supply the water to the bacteria removal element 8, and are formed of a material having an excellent water absorption property, for example, non-woven cloth made of acryl fiber, polyester fiber or the like, a sponge or the like.

One end portions (upper end portions) of the water absorption rods 19 are disposed so as to abut on a plate member 8C of an upper surface. Then, the rods extend through the bacteria removal element 8 in the vertical direction (the downward direction) from this one end portion, and then exit from the other end (the lower end) of the bacteria removal element 8, and the other end portions (the lower end portions) of the rods are constituted so as to protrude from'a bottom portion (the plate member 8C at the bottom surface) of the bacteria removal element 8. That is, the plate member 8C at the bottom surface is provided with holes (not shown) through which the water absorption rods 19 extend, and the other ends of the water absorption rods 19 protrude from the holes to further extend downwards. A length dimension of the water absorption rods 19 protruding from the bottom portion of the bacteria removal element 8 is smaller than a height dimension of the rods in the water storage tank 9 (the electrolytic tank tray 25), but is set to be sufficiently large. An edge portion of the hole of the plate member 8C at the bottom surface is provided with a holding portion 8D which holds the water absorption rods 19 and which has a substantially donut-like shape and which protrudes downwards, and the water absorption rods 19 are supported by the holding portion 8D.

The bacteria removal element 8 is detachably attached to the inside of the communication hole 30 formed at the receiving plane 1A of the main body 1. Here, a method of attaching the bacteria removal element 8 will be described. First, a packing 34 as a seal member is attached to the whole peripheral edge of the upper end of the engagement portion 30A formed at the periphery of the communication hole 30 of the receiving plane 1A, and an element receiver 35 is mounted from the upside of the packing. Furthermore, the bacteria removal element 8 integrated with the water absorption rods 19 is inserted into a space between the holding surface 8A of the first fixing plate 40 and the holding surface 8B of the second fixing plate from the upside of the element receiver 35, and the bottom surface of the plate member 8C of the bacteria removal element 8 is mounted on the upper surface of the element receiver 35. At this time, an outer peripheral surface of the holding portion 8D of the plate member 8C abuts on an inner peripheral surface of the packing 34, and the packing 34 is brought in close contact with the holding portion 8D. In consequence, the bacteria removal element 8 is mounted on the main body 1. In a state in which the bacteria removal element 8 is mounted on the main body 1, the communication hole 30 is closed with the bacteria removal element 8 via the packing 34 and the element receiver 35, and hermetically sealed, and the water storage tank 9 is therefore brought into a sealed or semi-sealed state. In this state, the water absorption rods 19 enter the water storage tank 9, and the other end portions of the rods are submerged into the electrolytic water.

On the other hand, the water supply tank 15 is a cartridge type tank for refilling the water storage tank 9 with the tap water. The water supply tank 15 is formed into such a shape so that the tank rises in the vertical direction from a circular opening 15A formed at a bottom surface thereof, then expands externally, and further rises in the vertical direction, and an upper end of the tank is closed with the upper surface 15B having a diameter larger than that of the opening 15A. Then, water is supplied inwardly from the opening 15A formed at the lower end of the tank, and the water storage tank 9 is refilled with the tap water from this opening 15A via a water passing port 64 formed at a cap receiver 62 described later.

Moreover, an outer peripheral surface of the water supply tank 15 on an opening 15A side is provided with a thread ridge 61 to be engaged with a thread groove (not shown) formed on an inner surface side of a cap 60. The cap 60 holds the water supply tank on the opening 15A side, and detachably attaches the water supply tank 15 to the communication hole 33 formed on the other end side of the receiving plane 1A of the main body 1 in the sealed manner together with the cap receiver 62 and packing 65, 66 as a seal member.

In a state in which the water supply tank 15 is attached to the lower end of the cap 60, a cylindrical water stop portion 60A which rises along an inner surface of the opening 15A of the water supply tank 15 is formed. On the upside of this water stop portion 60A, a water stop valve 72 is disposed. This water stop valve 72 is a valve device for avoiding water leakage from the water supply tank 15 in a case where the water supply tank is carried or the water supply tank 15 is attached to the main body 1. An outer diameter of this water stop valve 72 is set to be smaller than an inner diameter of the water supply tank 15 around the water supply tank 15, and larger than an inner diameter of the water stop portion 60A. A shaft component 73 of the water stop valve is attached to the center of the water stop valve 72, and a lower end of the shaft component is attached to the cap receiver 62.

Furthermore, in a usual state in which the water supply tank 15 is attached to the main body 1, the water stop valve 72 is positioned with a predetermined gap from the water stop portion 60A above the water stop portion 60A. Therefore, as shown by arrows in FIG. 3, the tap water in the water supply tank 15 passes through this gap to reach the opening 15A, and is supplied into the water storage tank 9 from this opening 15A via the water passing port 64 of the cap receiver 62.

In addition, in a state in which the water supply tank 15 is not attached to the main body 1, a spring member 75 attached to a lower end side of the shaft component 73 elongates, the water stop valve 72 is pulled downwards by the spring member 75 to move downwards, and abuts on the upper surface of the water stop portion 60A, so that the gap is closed to prohibit water supply from the water supply tank 15.

Moreover, in the state in which the water supply tank 15 is attached to the main body 1, the water supply from this water supply tank 15 into the water storage tank 9 is controlled by a valve device 70. The valve device 70 of the present embodiment is constituted of a float valve 71 which controls the water supply'from the water supply tank 15 in accordance with a water level of the electrolytic water in the water storage tank 9.

With regard to the float valve 71, a float to be constantly positioned on a water surface of the electrolytic water in the water storage tank 9 is attached to one end of a main body constituted of a plate member having a longitudinal shape, and the other end of the main body is tiltably supported by the bottom surface of the cap receiver 62. In consequence, the float valve 71 can tilt in the upward/downward direction in accordance with the water level of the electrolytic water in the water storage tank 9 where the float at the one end of the float valve 71 is positioned.

Moreover, in the valve device 70 having the above-mentioned constitution, when the electrolytic water in the water storage tank 9 is stored up to a predetermined water level above the other end portions (the lower end portions) of the water absorption rods 19, the water passing port 64 is closed with the upper surface of the main body of the float valve 71, and the water supply from the water supply tank 15 to the water storage tank 9 is securely prohibited. Furthermore, when the water level of the electrolytic water in the water storage tank 9 drops below the predetermined water level, the water passing port 64 is opened by a tilt operation of the float valve 71 to supply the water into the water storage tank 9. It is to be noted that the water level at which this float valve 71 opens the water passing port 64 is a water level at which the other end portions (the lower end portions) of the water absorption rods 19 are submerged into the electrolytic water in the water storage tank 9.

Furthermore, during such water supply from the water supply tank 15, in a case where the water level of the electrolytic water rises to such an extent that at least the other end portions (the lower end portions) of the water absorption rods 19 are positioned above the electrodes 17, 18 in the water storage tank 9, that is, the predetermined water level to prohibit the water supply is reached, the water passing port 64 is securely closed with the float valve 71 to prohibit the water supply from the water supply tank 15.

Since the water absorption rods 19 can constantly be submerged into the electrolytic water by the valve device 70 of the present embodiment in this manner, the supply of the electrolytic water to the bacteria removal element 8 can firmly be secured.

In addition, this float valve 71 is constituted so that when the main body 1 tilts, the water supply from the water supply tank 15 into the water storage tank 9 is securely prohibited. That is, when one end side of the main body 1 on the bacteria removal element 8 side tilts high, the other end side of the main body 1 on a water supply tank 15 side tilts low, and the water surface in the water storage tank 9 on the water supply tank 15 side rises, and needless to say, the float valve 71 closes the water passing port 64. However, the present float valve 71 is constituted so that even when the other end side of the main body 1 on the water supply tank 15 side tilts high and the water level of the opening 15A of the water supply tank 15 drops, the water supply into the water storage tank 9 can be prohibited.

That is, when the other end side of the main body 1 on the water supply tank 15 side and the water level of the opening 15A of the water supply tank 15 drops below the predetermined water level, the water in the water storage tank 9 moves toward the bacteria removal element 8, but it is assumed that the water storage tank 9 has the sealed structure or the semi-sealed structure (the semi-sealed structure in the present embodiment) as described above. Therefore, since the electrolytic water scarcely leaks from the water storage tank 9, a constant water level can be kept in the water storage tank 9 on the water supply tank 15 side. In consequence, the state in which the float valve 71 closes the water passing port 64 can be maintained.

According to the above constitution, it is possible to securely avoid a disadvantage that even when the water supply tank 15 side tilts high, water is excessively supplied from the water supply tank 15 to the water storage tank 9.

On the other hand, in the water storage tank 9 at the bottom surface of the receiving plane 1A at the center of the main body 1 provided with the communication hole 31 to which the electrodes 17, 18 are attached and the communication hole 32 disposed in parallel with this communication hole 31 and connected to the air passing tube 37, a partition wall 50 is disposed which separates an upper space around the electrodes 17, 18 from another part. The partition wall 50 extends in the vertical direction from the bottom surface of the receiving plane 1A of the main body 1 in the water storage tank 9, and a tip end of the partition wall is disposed in the electrolytic water stored in the water storage tank 9. The tip end is positioned with a sufficient gap from the upper surface of the electrolytic tank tray 25 constituting the bottom surface of the water storage tank 9 so that there is not any trouble in circulation of the electrolytic water.

In a space surrounded with the partition wall 50, one end of the air passing tube 37 connected to the communication hole 32 is also disposed together with the electrodes 17, 18. The air passing tube 37 is a gas release device for releasing a gas from the water storage tank 9 having the sealed structure or the semi-sealed structure. The air passing tube 37 of the present embodiment extends from the water storage tank 9 via one end thereof which opens (an inlet of the gas release device) in the space above the water surface of the electrolytic water of the water storage tank 9 surrounded with the partition wall 50 in the water storage tank 9, and passes around the air blower 20. Afterward, the other end of the tube opens (an outlet of the gas release device) in the vicinity of an upper end of the bacteria removal element 8 on a side opposite to the side panel 2R.

That is, when the electrodes 17, 18 are energized, the tap water in the water storage tank 9 is electrolyzed as described above to produce the sterile water (the electrolytic water) including hypochlorous acid (HC10), but a gas (hydrogen, oxygen or the like) is generated in the electrodes 17, 18 in a process to produce this electrolytic water. Here, when any gas release device is not disposed, the water storage tank 9 of the present invention has the sealed structure or the semi-sealed structure, and hence the generated gas is stored on the water surface of the electrolytic water in the water storage tank 9. Then, a pressure in the water storage tank 9 gradually increases owing to the generated and accumulated gas, so that the water storage tank 9 might be deformed or broken, and the stored electrolytic water might leak in the worst case.

To solve the problem, the air passing tube 37 is disposed as in the present invention, whereby the gas generated in the electrodes 17, 18 can be discharged from the water storage tank 9 via the air passing tube 37. Especially, peripheries of the electrodes 17, 18 are surrounded with the partition wall 50, separated from the other part, and especially separated from the space above the water surface of the electrolytic water of the water storage tank 9 outside the partition wall 50, whereby the gas generated in the electrodes 17, 18 gathers in the space above the water surface of the electrolytic water surrounded with the partition wall 50. Therefore, the opening of one end of the air passing tube 37 is disposed in this space, so that the air passing tube 37 can smoothly trap the gas generated in the electrodes 17, 18 to discharge the gas from the water storage tank 9 via the other end that opens in the vicinity of the upper end of the bacteria removal element 8 on the side opposite to the side panel 2R. In consequence, a disadvantage that the pressure in the water storage tank 9 increases owing to the generated gas can be eliminated in advance.

Especially, the opening (the outlet) of the other end of the air passing tube 37 is arranged in the vicinity of the bacteria removal element 8. Therefore, even when the main body 1 tilts and the electrolytic water in the water storage tank 9 enters the air passing tube 37 and is discharged from the opening of the other end, the electrolytic water is discharged into the bacteria removal element 8, and hence the water can be absorbed by the bacteria removal element 8. In consequence, a disadvantage that the electrolytic water leaks from the air bacteria removal device VW can be prevented as much as possible.

Next, an operation of the air bacteria removal device VW according to the present embodiment having the above-mentioned constitution will be described. First, when the power switch 13 of the operating section 10 formed at the top plate 2T of the main body cover 2 is pressed to turn on power supply, the electrodes 17, 18 start to be energized. In consequence, the tap water stored in the water storage tank 9 is electrolyzed to produce the electrolytic water including hypochlorous acid (an electrochemical treatment).

Moreover, simultaneously with the energizing of the electrodes 17, 18, the air blower 20 is started. In consequence, the air path is formed in which the air of the room (in the bacteria removal space) sucked from the suction port 4 successively passes through the filter 7, the air blower 20 and the bacteria removal element 8, and is discharged from the exhaust port 5. That is, the air in the room sucked from the suction port 4 passes through the gap between the front panel 2A of the main body cover 2 and the filter 7, and passes through the filter 7. At this time, dust included in the air is trapped by the filter 7. Since the filter 7 of the present embodiment has a deodorizing effect in addition to a dust collecting effect, odor is removed from the air when passing through the filter 7. The air from which the dust and the odor have been removed with the filter 7 is sucked into the air blower 20 from the fan air suction port 23A formed on the front surface side of the fan casing 23 in the axial direction in which the air blowing fan 21 rotates. Then, the air is discharged from the fan exhaust port 23B formed at the lower portion of the fan casing 23 on the one end side in the radial direction of the air blowing fan 21, and passed and supplied to the bacteria removal element 8 via the air duct 48 formed on the exhaust side of this fan exhaust port 23B.

The air supplied to the bacteria removal element 8 comes in contact with hypochlorous acid infiltrated in the bacteria removal element 8. Hypochlorous acid has a function of destroying and eliminating (removing) influenza virus surface protein (spike) which is indispensable for infection of influenza in a case where, for example, influenza virus invades the air in the room. In a case where this protein is destroyed, the influenza virus does not bond to a receptor of an infectee, which is necessary for the infection of the virus. In consequence, the infection is inhibited. The air in the room is supplied to the bacteria removal element 8 and brought into contact with the electrolytic water supplied to the bacteria removal element 8 in this manner, so that the bacteria can be removed from the air by hypochlorous acid included in the electrolytic water.

Moreover, the air which has passed through the bacteria removal element 8 and from which the bacteria has been removed is discharged to the outside (into the room) from the exhaust port 5 of the side panel 2R disposed on the one end side of the bacteria removal element 8.

According to the air bacteria removal device VW of the present invention described above in detail, the electrolytic water electrolyzed, generated and stored in the water storage tank 9 by the energizing of the electrodes 17, 18 is quickly supplied to the bacteria removal element 8 via the water absorption rods 19 by use of a capillary phenomenon, and the electrolytic water is brought into contact with the air passed from the air blower 20 in the bacteria removal element 8, so that the bacteria can efficiently be removed from the air.

In this case, since the electrolytic water stored in the water storage tank 9 can be supplied to the bacteria removal element 8 without disposing any electrolytic water drip means such as a special pump mechanism as in the conventional example, the structure can be simplified, and the device can be miniaturized. Since the pump mechanism is not required, any power for driving a pump is not consumed, and hence power consumption can be reduced.

Moreover, the water storage tank 9 in which the electrolytic water is stored is constituted by attaching the tank to the main body 1 via the packing 26 at the upper end opening edge of the electrolytic tank tray 25 in the sealed manner, and the communication holes 30, 31, 32 and 33 formed at the receiving plane 1A of the main body 1 are similarly sealed and closed with the bacteria removal element 8 integrated with the water absorption rods 19, the electrode fixing plate 54 which fixes the electrodes 17, 18, the air passing tube 37 and the water supply tank 15, respectively. Therefore, the water storage tank 9 has the sealed structure or the semi-sealed structure, sealability of the water storage tank 9 is secured, and the water does not easily leak from the structure.

Especially, in a conventional circulation type device in which the electrolytic water brought into contact with the bacteria removal element 8 is returned into the water storage tank 9, a structure has to be constituted in which at least the electrolytic water excessively supplied to the gas-liquid contact member can be collected. Therefore, there has been a problem that the structure is complicated, the device is enlarged and costs increase. However, in the present invention, since the electrolytic water in the water storage tank 9 is supplied using the capillary phenomenon, unlike the conventional example, the electrolytic water supplied to the bacteria removal element 8 does not have to be collected, so that the path of the water and the structure can be simplified to miniaturize the device. Furthermore, the water storage tank 9 can easily be formed into the sealed structure, and the sealability of the water storage tank 9 can further be improved. In consequence, the present invention can be mounted in a vehicle such as a car or a train. In a case where the air bacteria removal device of the present invention is mounted in the vehicle in this manner and the air bacteria removal device is used on conditions that the device tilts during running along a slope or the like, such a structure is more effective.

Furthermore, the bacteria removal element 8 and the water absorption rods 19 are integrated and detachably attached to the main body 1, so that the bacteria removal element 8 and the water absorption rods 19 can freely be removed from the main body. The dust attached to the bacteria removal element 8 and the water absorption rods 19 from the passed air and a scale in the electrolytic water can be cleaned. After the cleaning, the element and the rods can easily be mounted on the main body 1. In consequence, daily maintenance is easily performed.

Especially, the filter 7 is disposed in the air path on the windward side from the bacteria removal element 8, the dust in the air is removed, and the air is then supplied to the bacteria removal element 8, so that the attachment of the dust to the bacteria removal element 8 can be eliminated or inhibited. Furthermore, since the filter 7 of the present embodiment also has the deodorizing function, the odor in the air can be removed.

Furthermore, as in the present embodiment, the air blower 20 is constituted of the sirocco fan for sucking the air from the direction of the axis around which the air blowing fan 21 rotates to discharge the air in the radial direction, whereby the bacteria removal element 8 is arranged in the axial direction around which the air blowing fan 21 of the air blower 20 rotates. Therefore, the water storage tank 9 also has a longitudinal shape, and the whole device can be slimmed. In consequence, while the miniaturization of the device is achieved, it is possible to secure a series of operations of sucking the outside air (the air in the room) to blow the air to the bacteria removal element 8, refilling the water storage tank 9 with the tap water from the water supply tank 15, and electrolyzing the water to supply the water to the bacteria removal element 8.

## Claims

1. An air bacteria removal device in which an air-liquid contact member formed of a material less reactive with electrolytic water brings the electrolytic water into contact with passed air to remove bacteria, the device comprising:
a main body including a water storage section having a sealed or semi-sealed structure in which the electrolytic water is stored; and
a water absorption member which supplies the electrolytic water from the water storage section to the air-liquid contact member by use of a capillary phenomenon,
wherein the air-liquid contact member and the water absorption member are integrated and detachably attached to the main body, the water absorption member attached to the main body enters the water storage section, and is submerged into the electrolytic water, and the water storage section is brought into a sealed or semi-sealed state.

2. An air bacteria removal device in which an air-liquid contact member formed of a material less reactive with electrolytic water brings the electrolytic water into contact with passed air to remove bacteria, the device comprising:
a main body including a water storage section having a sealed or semi-sealed structure in which the electrolytic water is stored; and
air blow means for passing air through the air-liquid contact member,
wherein the electrolytic water is supplied from the water storage section to the air-liquid contact member by use of a capillary phenomenon, and dust collection means and/or deodorizing means is disposed in an air path in which the air is passed through the air-liquid contact member from the air blow means.

3. An air bacteria removal device in which an air-liquid contact member formed of a material less reactive with electrolytic water brings the electrolytic water into contact with passed air to remove bacteria, the device comprising:
a main body including a water storage section having a sealed or semi-sealed structure in which the electrolytic water is stored;
electrodes which are positioned in the water storage section and which are energized to electrochemically treat water including at least chloride ions in the water storage section, thereby producing the electrolytic water;
a partition wall which separates an upper space around the electrodes from another part; and
a gas release device which discharges a gas from the space on an inner side of the partition wall,
wherein the electrolytic water is supplied from the water storage section to the air-liquid contact member by use of a capillary phenomenon.

4. An air bacteria removal device in which an air-liquid contact member formed of a material less reactive with electrolytic water brings the electrolytic water into contact with passed air to remove bacteria, the device comprising:
a main body including a water storage section having a sealed or semi-sealed structure in which the electrolytic water is stored;
electrodes which are positioned in the water storage section and which are energized to electrochemically treat water in the water storage section, thereby producing the electrolytic water; and
a gas release device which discharges a gas from the water storage section,
wherein the electrolytic water is supplied from the water storage section to the air-liquid contact member by use of a capillary phenomenon, and an outlet of the gas release device is arranged in the vicinity of the air-liquid contact member.

5. An air bacteria removal device in which an air-liquid contact member formed of a material less reactive with electrolytic water brings the electrolytic water into contact with passed air to remove bacteria, the device comprising:
a main body including a water storage section having a sealed or semi-sealed structure in which the electrolytic water is stored;
electrodes which are positioned in the water storage section and which are energized to electrochemically treat water including at least chloride ions in the water storage section, thereby producing the electrolytic water;
a water supply tank which is detachably attached to the main body and which refills the water storage section with the water including at least the chloride ions; and
a valve device which prohibits water supply from the water supply tank in a case where the main body tilts,
wherein the electrolytic water is supplied from the water storage section to the air-liquid contact member by use of a capillary phenomenon.

6. An air bacteria removal device in which an air-liquid contact member formed of a material less reactive with electrolytic water brings the electrolytic water into contact with passed air to remove bacteria, the device comprising :
a main body including a water storage section having a sealed or semi-sealed structure in which the electrolytic water is stored;
electrodes which are positioned in the water storage section and which are energized to electrochemically treat water including at least chloride ions in the water storage section, thereby producing the electrolytic water;
a water supply tank which is detachably attached to the main body and which refills the water storage section with the water including at least the chloride ions; and
air blow means for passing air through the air-liquid contact member,
wherein the electrolytic water is supplied from the water storage section to the air-liquid contact member by use of a capillary phenomenon, and the air is introduced from the outside by the air blow means, brought into contact with the air-liquid contact member and then discharged to the outside.

7. The air bacteria removal device according to claim 6, further comprising:
a water absorption member having one end portion which comes in contact with the air-liquid contact member and the other end portion submerged into the electrolytic water in the water storage section to supply the electrolytic water to the air-liquid contact member by use of the capillary phenomenon; and
a float valve device which controls the water supply from the water supply tank,
wherein when a water level of the electrolytic water rises above a water level at which at least the other end portion of the water absorption member is submerged into the electrolytic water in the water storage section, the float valve device prohibits the water supply from the water supply tank.

8. The air bacteria removal device according to claim 6 or 7, wherein the air-liquid contact member is arranged on one end portion of the main body, the water supply tank is arranged on the other end portion of the main body, the water storage section is constituted from the one end portion to the other end portion of the main body, the electrodes are arranged in the water storage section between the water supply tank and the air-liquid contact member, the air blow means is arranged on the main body between the air-liquid contact member and the water supply tank, and air is sucked from an axial direction around which the air blow means rotates to discharge the air in a radial direction.

9. The air bacteria removal device according to any one of claims 1 to 8, wherein the air-liquid contact member is hydrophilically treated.
